(19) Europäisches Patentamt
European Patent Office
Office européen des brevets

(11) Veröffentlichungsnummer: **0 393 231 B1**

(12) **EUROPÄISCHE PATENTSCHRIFT**

(45) Veröffentlichungstag der Patentschrift:
18.12.91 Patentblatt 91/51

(51) Int. Cl.⁵: **A61L 11/00**

(21) Anmeldenummer: 89108235.6

(22) Anmeldetag: 08.05.89

(54) **Vorrichtung und Verfahren zur Behandlung von medizinischen Sonderabfällen.**

(30) Priorität: 19.04.89 DE 3912751

(43) Veröffentlichungstag der Anmeldung:
24.10.90 Patentblatt 90/43

(45) Bekanntmachung des Hinweises auf die
Patenterteilung:
18.12.91 Patentblatt 91/51

(84) Benannte Vertragsstaaten:
NL SE

(56) Entgegenhaltungen:
Keine

(73) Patentinhaber: VETCO SANITEC GMBH
Maschweg 5
W-3100 Celle (DE)

(72) Erfinder: Göldner, Helmut
Bremer Str. 4
W-3070 Nienburg (DE)
Erfinder: Kamm, Reinhold, Dipl.-Ing.
Schaffeld 12
W-3101 Wienhausen (DE)
Erfinder: Leinski, Heinz, Dipl.-Kfm.
Fuhrbergsweg 4
W-3101 Wathlingen (DE)

(74) Vertreter: Sparing - Röhl - Henseler
Patentanwälte
Rethelstrasse 123 Postfach 14 02 68
W-4000 Düsseldorf 1 (DE)

## Beschreibung

Die Erfindung betrifft eine Vorrichtung und ein Verfahren zur Behandlung von medizinischen Sonderabfällen nach dem Oberbegriff der Ansprüche 1 und 23.

In Krankenhäusern, medizinischen Laboratorien, Arztpraxen und sonstigen Einrichtungen des Gesundheitswesens entsteht täglich eine große Menge an potentiell infektiösem Müll, wie beispielsweise Einwegmaterial, Verbände, Spritzen und Kanülen, die mit infektiösen Patienten in Berührung gekommen sind, und an infektiösem Müll, wie beispielsweise mit Bakterien, Viren oder Sporen mikrobiell kontaminierte Abfälle. Diese medizinischen Sonderabfälle bedürfen bei der Lagerung und dem Transport besonderer Maßnahmen zur Infektionsverhütung.

Als Entsorgungsmaßnahme bekannt ist daher das Sammeln dieses Sondermülls in verschlußsicheren Einwegbehältern mit anschließender Verbrennung in Spezialanlagen. Die Infektionsgefahren für das medizinische Personal und die Risiken bei der Beförderung zur Verbrennungsanlage mittels Straßenfahrzeugen sind jedoch groß. Zudem betragen die Verbrennungskosten für den Sondermüll ein Vielfaches der Kosten für Hausmüll.

Eine andere bekannte Entsorgungsmaßnahme ist die Desinfektion infektiöser Abfälle und damit ihre Aufbereitung zu hausmüllähnlichen Abfällen, die wie normaler Hausmüll abgefahren und beseitigt oder nach einer Sortierung dem Recycling zugeführt werden können. Aus der DE-OS 3317300 ist hierzu ein Behälter zur Aufnahme von spezifischen Krankenhausabfällen bekannt, der nach einem Auffüllen und Einbringen eines Desinfektionsmittels entweder in eine Mikrowellenkammer gestellt werden kann oder selbst mit Mikrowellensendern ausgerüstet ist, so daß der Sondermüll durch eine chemothermische Abtötung der Mikroorganismen desinfiziert wird. Dieser Behälter läßt nur eine chargenweise Behandlung des Sondermülls in geringem Umfang zu, wobei vorbereitende Abfallbeseitigungsschritte, wie das Zerkleinern des Sondermülls, keine ausreichende Infektionsverhütung gewährleisten. Aus der Sicht der Umwelthygiene und der Toxizität von Desinfektionswirkstoffen sind derartige chemische Verfahren außerdem nur bedingt einsetzbar.

Die DE-PS 3505570 zeigt eine Vorrichtung zur Behandlung von Infektionsmüll mit Hilfe von Mikrowellen, bei der eine Desinfektion von Infektionsmüll in einer kontinuierlich arbeitenden Entsorgungsanlage durchgeführt wird, um das Infektionsrisiko durch Freigabe von Infektionskeimen, Bakterien usw. so gering wie möglich zu halten. Diese kompakte Entsorgungsanlage umfaßt eine Schleusenkammer, eine darin angeordnete Sprühvorrichtung zum Befeuchten des Mülls mit Wasser und gegebenenfalls einem Zusatz von Desinfektionsmittel, einen Müllzerkleinerer und eine Mikrowellenkammer. Die Mikrowellenkammer ist als zumindest teilweise mikrowellendurchlässiges Durchgangsrohr ausgebildet, entlang dem mehrere benachbart zueinander angeordnete Mikrowellensender vorgesehen sind. Mittels einer Fördereinrichtung wird der zerkleinerte und befeuchtete Müll durch die Mikrowellenkammer hindurchbewegt, wobei die Mikrowellenstrahlung zu einer starken Erwärmung des Mülls führt. Die Verweildauer des Mülls in der Mikrowellenkammer wird über die Temperatur gesteuert, wobei abhängig vom Material eine Temperatur von ca. 135°C und höher bis maximal 200°C erreicht werden muß. Die Verweildauer des Mülls in der Mikrowellenkammer beträgt dann einige Minuten. Eine derartige Entsorgungsanlage hat für Durchlaufzeiten mit vertretbarem wirtschaftlichem Aufwand zu keiner absolut zuverlässigen Desinfektion geführt, was einen vermehrten Einsatz von Desinfektionsmitteln erforderlich machte.

Aufgabe der Erfindung ist es daher, eine Vorrichtung und ein Verfahren der genannten Art zu schaffen, die eine sichere und zuverlässige Behandlung zur Entsorgung von medizinischen Sonderabfällen in wirtschaftlicher und umweltfreundlicher Weise erlauben.

Diese Aufgabe wird gemäß dem kennzeichnenden Teil der Ansprüche 1 und 23 gelöst.

Hierdurch werden eine Vorrichtung und ein Verfahren zur Behandlung von medizinischen Sonderabfällen geschaffen, die eine Verminderung der Keimzahl durch thermische Inaktivierung erlauben. Eine schnelle Aufheizung des zerkleinerten, feuchten Mülls in der Mikrowellenkammer und ein Halten des Mülls auf wenigstens einer wählbaren Mindesttemperatur während einer Mindestverweildauer erlauben eine Anpassung der Behandlung an die Ausgangskeimzahl und die vorhandenen Keimarten, damit es unter dem Einfluß der feuchten Hitze zu einer mindestens teilweisen Denaturierung von Eiweißen und Nukleinsäuren in den Bakterien, Pilzen und Viren kommt. Diese Schädigung ist irreversibel und führt zu einer sicheren Ausschaltung der Wachstums- und Vermehrungsfunktionen. Eine gezielte, partielle Keimtötung bzw. Inaktivierung und damit Eliminierung von Krankheitserregern, die sich in oder an den Müllpartikeln befinden, kann so durch eine rein thermische Desinfektion erreicht werden. Infektiöser Müll wird schnell und mit hohem Wirkungsgrad dekontaminiert, wobei der höhere Wärmegehalt von feuchter Luft genutzt wird.

Dabei kann die Vorrichtung stationär aufgestellt oder mobil genutzt werden. In Gebieten mit Krankenhäusern und anderen Einrichtungen des Gesundheitswesens, deren Müllaufkommen gering ist, kann die Vorrichtung als Kraftfahrzeugaufsatz in einem Container untergebracht sein, mit dem in regelmäßigen Zeitabständen die Abfallsammelstellen der Krankenhäuser angefahren werden. Durch den Einsatz dieses dezentralen Entsorgungssystems werden die medizinischen Abfälle umweltschonend und preisgünstig entsorgt. Der Transport von infektiösen Abfällen über die Straße und die daraus resultierende Gefahr von Infektionen wird vermieden. Durch die nicht mehr erforderlichen Einwegsammelbehälter reduziert sich das Volumen des Abfalls erheblich, so daß sich auch die Kosten der Abfallbeseitigung verringern.

In der Mikrowellenkammer wird das feuchte bzw. befeuchtete Müllgranulat durch die Mikrowellen direkt erwärmt, wodurch ein schnelles Aufheizen des Granulats bis zum Siedepunkt des Wassers erreicht wird. Die damit verbundene Dampfbildung und Dampfdurchströmung steigert die Aufheizung durch eine indirekte Erwärmung. Speziell bei müllspezifischem Material mit schlechter Wärmeleitfähigkeit, wie z.B. Kunststoffen, ergibt sich so eine wesentlich schnellere und wirtschaftlichere Erwärmung des eingefüllten Mülls. Zur Verbesserung einer gleichmäßigen Temperaturverteilung über den Querschnitt einer gewissen Schichtdicke kann die Fördereinrichtung als Mikrowellenfeldverteiler in Form einer wellenlosen Förderschnecke aus Metall ausgebildet sein. Diese Förderschnecke bewirkt folglich nicht nur eine gute Durchmischung des aufzuheizenden Mülls, sondern löst auch Mehrfachreflexionen aus, so daß die einzelnen Erwärmungsunterschiede durch direkte und indirekte Erwärmung ausgeglichen werden. Eine Mikrowellenreflexion von den Wandungen der Mikrowellenkammer kann durch eine Ausbildung derselben als metallischer U-Trog erreicht werden. Eine verbesserte Durchmischung kann desweiteren durch eine geneigte Aufstellung der Mikrowellenkammer und das dadurch bedingte Zurückfallen des Granulats erzielt werden. Zur Vermeidung von Wärmeverlusten kann die Mikrowellenkammer schließlich thermisch isoliert sein und gegebenenfalls eine Stützheizung aufweisen, die elektrisch, mit Thermoöl oder mit Heißdampf betreibbar ist.

Während die dichte Mikrowellenfeldverteilung in einer ersten Stufe der Behandlung für ein schnelles Aufheizen genutzt wird, erfolgt in einer zweiten Stufe das Halten einer Einwirktemperatur oder -bereiches zur vollständigen Ausschaltung der pathogenen Keime. Zur Aufrechterhaltung einer Mindesttemperatur ist die Temperaturhaltekammer vorzugsweise von einer Heizeinrichtung ummantelt. Die Bewegung des Mülls durch die Temperaturhaltekammer während der wählbaren Einwirkzeit kann zwangsweise mittels einer Fördereinrichtung oder unter Wirkung der Schwerkraft erfolgen. Zur Überwachung der Temperatur am Eingang und Ausgang der Temperaturhaltekammer können Temperaturfühler vorgesehen sein.

Die Vorrichtung kann für eine halb- oder vollautomatische Betriebsweise eine programmierbare Zentralsteuereinheit aufweisen, die eine Überwachung und Steuerung der drei Bereiche Eingabe- und Zerkleinerung, Aufheizung und Temperaturhaltung vornimmt und eine jeweilige Anpassung der Betriebsweise auf die Art und Menge des zu entsorgenden Sondermülls erlaubt.

Weitere Ausgestaltungen der Erfindung sind der nachfolgenden Beschreibung und den Unteransprüchen zu entnehmen.

Die Erfindung wird nachstehend anhand der in den beigefügten Abbildungen dargestellten Ausführungsbeispiele näher erläutert.

Fig. 1            zeigt einen Längsschnitt eines erstes Ausführungsbeispiels einer Vorrichtung zur Behandlung von medizinischen Sonderabfällen,

Fig. 2a und 2b     zeigen jeweils einen halben Querschnitt einer Mikrowellenkammer der Vorrichtung gemäß Fig. 1,

Fig. 3            zeigt ein Blockdiagramm von Funktionseinheiten der Vorrichtung gemäß Fig. 1,

Fig. 4            zeigt einen Längsschnitt einer anderen Ausführungsform des Eingabe- und Zerkleinerungsbereiches,

Fig. 5            zeigt einen Längsschnitt eines zweiten Ausführungsbeispiels einer Vorrichtung zur Behandlung von medizinischen Sonderabfällen,

Fig. 6            zeigt einen Längsschnitt eines drittes Ausführungsbeispiels einer Vorrichtung zur Behandlung von medizinischen Sonderabfällen,

Fig. 7            zeigt einen Längsschnitt einer mobilen Ausführungsform einer Vorrichtung zur Behandlung von medizinischen Sonderabfällen.

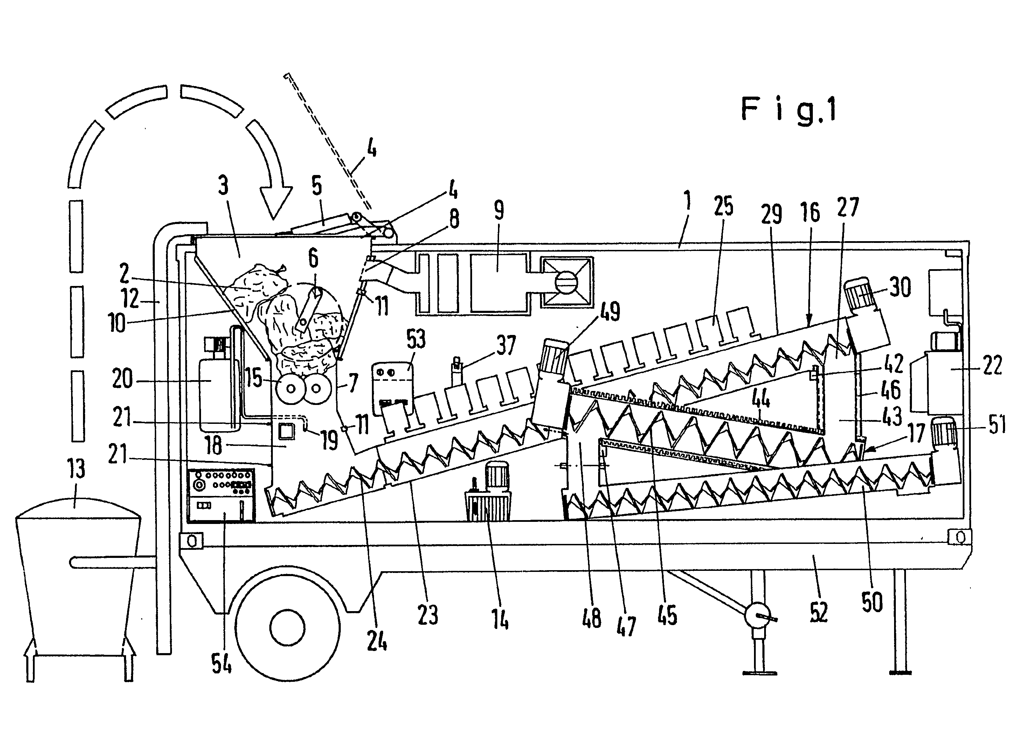

Figur 1 zeigt ein erstes Ausführungsbeispiel einer Vorrichtung zur Behandlung von medizinischen Sonderabfällen in einem Container 1, die einen Eingabeabschnitt, einen Behandlungsabschnitt und einen Ausförderungsabschnitt zu einer kompakten Entsorgungsanlage integriert.

Der Eingabeabschnitt umfaßt zur Aufnahme der zu behandelnden Müllgegenstände 2 zunächst einen Einfüllraum 3, der als Trichter ausgebildet und über einen Deckel 4 fluiddicht verschließbar ist. Das Öffnen und Schließen des Deckels 4 erfolgt über Hydraulikzylinder 5, durch die der Deckel 4 auf dem Dach des Containers 1 befestigt ist. Innerhalb des Einfüllraumes 3 ist eine als Drehschwinge 6 ausgebildete Eindrückvorrichtung angeordnet, die den eingefüllten Müll 2 vorzerkleinert und einem Müllzerkleinerer 7 zuführt. Angetrieben wird die Drehschwinge 6 von einem Getriebemotor. Ein oder mehrere Absaugschlitze 8 einer Absauganlage 9 sind in den Seitenwandungen 10 des Einfüllraumes 3 angeordnet und bilden einen Absaugschleier, der die durch Öff-

3

nen des Dekkels 4 eventuell hochgezogenen Luftkeime absaugt. Im allgemeinen bleibt die Filteranlage 8 bei geöffnetem Deckel 4 in Betrieb, um ein Austreten von Keimen aus dem Einfüllraum 3 zu verhindern. Vorzugsweise umfaßt die Filteranlage 9 einen Vorfilter und einen Hochleistungsschwebstoffilter. Der Trichter mit Deckel 4 und Absauganlage 9 geben dem Einfüllraum 3 die Funktion einer Abfallschleuse. Desweiteren ist ein Injektionsanschluß 11 mit zugeordnetem Ventil in die Seitenwandung 10 eingelassen, damit bei Stillstand, Schichtende sowie Reparatur- und Wartungsarbeiten Heißdampf für eine Dekontaminierung in die Entsorgungsanlage eingeleitet werden kann.

Damit sich keine Keime an den Seitenwandungen 10 niederschlagen, sind diese noch oberflächenbeheizbar unter Verwendung einer Stützheizung. Die Seitenwandungen 10 werden auf eine Temperatur über 100°C erwärmt, vorzugsweise auf Temperaturen zwischen 105°C und 140°C.

Die Beschickung mit Abfallgegenständen 2 kann von Hand oder automatisch vorgenommen werden über eine Hub- und Kippvorrichtung 12, die Abfallbehälter 13 aufnimmt und in den Einfüllraum 3 entleert. Die Hub- und Kippvorrichtung 12 kann hierzu an der Heckseite des Containers 1 angeordnet sein und bewegt einen oder mehrere Abfallbehälter 13 für den jeweiligen Beschickungsvorgang in Pfeilrichtung. Bei den Abfallbehältern 13 handelt es sich vorzugsweise um 120 l bis 1100 l Behälter. Zur Betätigung der Hub- und Kippvorrichtung 12 ist eine Hydraulikanlage 14 vorgesehen. In die Hub- und Kippeinrichtung 12 kann eine Wägeeinrichtung integriert sein, die das Gewicht je Abfallbehälter 13 bestimmt und gegebenenfalls elektronisch aufzeichnet.

Der ebenfalls zu dem Eingabeabschnitt gehörende Müllzerkleinerer 7 besteht aus einem Schneidwerk mit zwei gegenläufigen Messerwellen 15, in die Schneidkörper und Mitnehmer eingeschoben sind. Die Schneidkörper sind so gestaltet, daß eine Granulierung des mit Hilfe der Drehschwinge 6 zugeführten Abfallmaterials erreicht wird. Gleichzeitig erfolgt eine Durchmischung des Mülls. Für den Antrieb des Müllzerkleinerers 7 ist ein steuerbarer Elektromotor vorgesehen.

Der Behandlungsabschnitt besteht aus einer Mikrowellenkammer 16 und einer Temperaturhaltekammer 17. Die Verbindung zwischen dem Eingabeabschnitt und dem Behandlungsabschnitt schafft ein Übergabetrichter 18, der an den Ausgang des Müllzerkleinerers 7 und den Eingang der Mikrowellenkammer 16 lösbar befestigt ist. Vorzugsweise ist der Übergabetrichter 18 angeflanscht. In den Übergabetrichter 18 ist ein Sprühkopf 19 eingesetzt, der an einen mit einer Pumpe versehenen Wassertank 20 angeschlossen ist aber auch an ein externes Wassernetz anschließbar ist. Die Sprüheinrichtung dient zum gezielten Einsprühen von Wasser für eine gleichmäßige Befeuchtung des von dem Müllzerkleinerer 7 erzeugten Granulats für die nachfolgende Behandlung. Das Sperren und Freigeben der Wasserwege erfolgt vorzugsweise durch Magnetventile. Sprühzeit und Pausenzeit können in Abhängigkeit vom Feuchtigkeitsgrad des eingefüllten Mülls über einen Taktgeber variiert werden.

Der Übergabetrichter 18 wird als Zwischenspeicher für das Granulat genutzt, da der Zerkleinerer 7 im allgemeinen mehr Granulat anbietet, als die Mikrowellenkammer 16 übernehmen kann. Zur Überwachung des Füllgrades des Übergabetrichters 18 ist im Bereich des Eingangs und des Ausgangs ein Füllstandssensor 21 für einen minimalen und maximalen Füllstand angeordnet. Der Elektromotor des Müllzerkleiners 7 wird vorzugsweise so gesteuert, daß der Füllstand im Übergabetrichter 18 immer zwischen dem minimalen und maximalen Füllstand pendelt. Über die Füllstnadssensoren 21 kann folglich der Zerkleinerungsvorgang geregelt werden. Schließlich kann der Übergabetrichter noch einen weiteren Injektionsanschluß 11 für das Einleiten des Heißdampfes für die Dekontaminierung der entleerten Entsorgungsanlage aufweisen. Zur Beaufschlagung dieser Injektionsanschlüsse 11 ist ein Dampferzeuger 22 vorgesehen.

Im Behandlungsabschnitt dient die Mikrowellenkammer 16 zur Aufheizung des zerkleinerten und feuchten, gegebenenfalls befeuchteten Mülls in einem Durchlaufverfahren mit einer wählbaren Fördergeschwindigkeit und Schichtdicke des zerkleinerten Mülls. Die Mikrowellenkammer 16 besteht aus einem kanalförmigen Durchgangsgehäuse 23, in dem eine Fördereinrichtung 24 angeordnet ist. Längs des Durchgangsgehäuses 23 ist ein zentraler Mikrowellensender mit einem Hohlleitersystem oder sind mehrere Mikrowellensender 25 dicht benachbart nebeneinander angeordnet. Zur Einkopplung der Mikrowellenstrahlung weist das Durchgangsgehäuse 23 Eintrittsöffnungen auf oder besteht in diesen Bereichen aus mikrowellendurchlässigem Material. Die Mikrowellensender 25 können an mehreren Seiten des Durchgangsgehäuses 23 angeordnet sein. Unter der Einwirkung der Mikrowellen wird das durchlaufende Granulat durch innere Erhitzung und verdampfter Feuchtigkeit aufgeheizt. Zum Auffangen der verdampfenden Feuchtigkeit bilden das Durchgangsgehäuse 23 und die daran befestigten Mikrowellensender 25 einen abgedichteten Behandlungsraum. Als Fördereinrichtung 24 kann beispielsweise eine Förderschnecke, ein Förderband oder ein Förderstempel verwendet werden. Die Fördereinrichtung 24 entnimmt den zerkleinerten Müll dem Übergabetrichter 18 und befördert diesen mit einer einstellbaren Geschwindigkeit zum Ausgang 27 der Mikrowellenkammer 16. Die Durchmischung des geförderten Granulats kann für eine gleichmäßige Bestrahlung und einen guten Wärmeaustausch durch ein Aufstellen der Mikrowellenkammer 16 mit einer Steighöhe verbessert werden. Da die Mikrowellenkammer stets nur teilweise gefüllt ist, vorzugsweise 2/3, fällt das Müllgranulat so immer teilweise wieder zurück. Der Neigungswinkel liegt

4

vorzugsweise zwischen 10° und 50°.

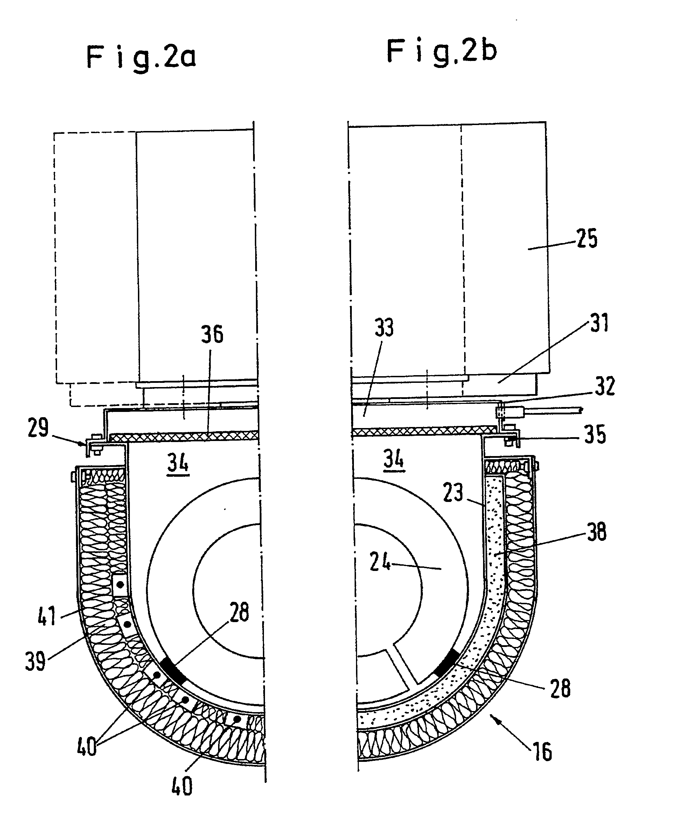

In den Figuren 2a und 2b ist der Aufbau der Mikrowellenkammer 16 im einzelnen dargestellt. Das Durchgangsgehäuse 23 ist als U-förmiger Trog ausgebildet und die Fördereinrichtung 24 besteht aus einer offenen wellenlosen Förderspirale, die in dem U-förmigen Trog rotiert. Trog und Förderspirale bestehen aus Metall, vorzugsweise Edelstahl, und zusätzliche Verschleißschienen 28 aus einem weicheren Material sind vorgesehen, auf denen die Förderspirale läuft. Fluiddicht geschlossen wird der Trog 23 mittels eines Trogdeckels 29, auf dem die Mikrowellensender 25 befestigt sind. Im Bereich der Mikrowellensender 25 kann die Abdeckfunktion des Trogdeckels 29 auch von den Mikrowellensendern 25 mit angeschlossenem Leitersystem übernommen werden, wie nachstehend noch erläutert wird. Die Trogabmessungen richten sich nach der gewünschten Schichtdicke in der Mikrowellenkammer 16 und der Durchsatzmenge sowie Durchsatzgeschwindigkeit. Die Förderspirale hat durch des Fehlen einer Welle einen großen freien Querschnitt, wodurch die Verstopfungs- und Pfropfenbildungsgefahr minimiert wird. Desweiteren wirkt die Förderspirale als dreidimensionaler Feldverteiler der eingekoppelten Mikrowellen, wodurch das zu behandelnde Material besser von der Mikrowellenstrahlung erreicht wird. Angetrieben wird die Förderspirale von einem Motor 30, dessen Drehzahl steuerbar ist.

Die Aufheizung in der Mikrowellenkammer 16 wird durch zwei unterschiedliche Wärmeeinspeisungen während des Materialdurchlaufes erzielt. Eine erste Wärmeeinspeisung erfolgt durch die Mikrowellensender 25, von denen hier 12 nebeneinander angeordnet sind, deren Anzahl je nach Leistungsstärke allerdings zwischen 1 und 20 liegen kann. Die von den einzelnen Mikrowellensendern 25 erzeugte Mikrowellenenergie wird jeweils über einen Hohlleiter 31 und Leitbleche 32, die eine Resonanzkammer 33 bilden, in den Behandlungsraum 34 der Mikrowellenkammer 16 eingekoppelt. Über lösbare Befestigungseinrichtungen 35 sind die Leitbleche 32 an dem Trog 23 befestigt. Die Resonanzkammern 33 sind zur Erzeugung einer dichten Mikrowellenfeldverteilung unmittelbar nebeneinander angeordnet. Zur Unterbindung des Eintretens von Müllpartikeln und Feuchtigkeit in den Hohlleiter 31 und die Resonanzkammer 33 ist der Behandlungsraum 34 kopfseitig über Scheiben 36 aus einem mikrowellendurchlässigen Material. wie beispielsweise Polytetrafluorethylen (PTFE), abgedeckt. Die Einschaltzeit der Mikrowellensender 25 ist steuerbar. Die elektrische Versorgung kann über einen Steckeranschluß 37 (siehe Figur 1) erfolgen. Die Einschaltzeiten werden so gewählt, daß die Mikrowellen das Granulat auf oder über eine wählbare Mindesttemperatur erhitzen, damit die gewünschte thermische Behandlung, beispielsweise eine Desinfektion, durchgeführt werden kann. Zur Gewährleistung einer Aufheizung auf wenigstens die Mindesttemperatur erfolgt eine automatische Anpassung der Fördergeschwindigkeit und der Füllstände.

Eine zweite Wärmeeinspeisung dient zur Stützung der durch die Mikrowellen erzeugten Aufheizung. Hierzu ist der Trog 23 von einer Heizeinrichtung umgeben. Gemäß Figur 2a besteht die Heizeinrichtung aus elektrischen Heizspiralen 40, die vorzugsweise direkt an der Wandung des Trogs 23 angeordnet sind. Gemäß Figur 2b besteht die Heizeinrichtung aus einem doppelwandigen Teilmantel 38 für ein Wärmeträgermedium, wie beispielsweise Thermoöl oder Heißdampf, der ebenfalls vorzugsweise direkt an die Wandung des Trogs 23 angeordnet ist. Bei beiden Ausgestaltungen ist anschließend eine Wärmeisolierung 39 installiert, die über eine Abdeckung 41 außenseitig abgeschirmt ist. Diese zweite Wärmeeinspeisung kann aus verschiedenen Heizkreisen bestehen, damit einzelnen Bereichen der Mikrowellenkammer 16 eine gezielte kontinuierliche Wärmemenge zugeführt werden kann. Die erreichte Granulattemperatur wird im Bereich des Eingangs 43 der Temperaturhaltekammer 17 über einen Meßfühler 42 ermittelt. Zur thermischen Desinfektion eines feuchten Mediums liegt die Mindesttemperatur über 95 °C und beträgt vorzugsweise 98°C bis 102°C.

An den Ausgang 27 der Mikrowellenkammer 16 ist der Eingang 43 der Temperaturhaltekammer 17 angeschlossen. Die Temperaturhaltekammer 17 besteht aus einem kanalförmigen Durchgangsgehäuse 44, in dem der Temperaturvorgang des Granulats in einem Durchlaufverfahren erfolgt. Mittels einer Fördereinrichtung 45 wird das von der Fördereinrichtung 24 der Mikrowellenkammer 16 antransportierte erhitzte Granulat übernommen und während einer wählbaren Mindestverweildauer durch die Temperaturhaltekammer 17 hindurchbewegt. Während dieser Zeit wird das Granulat auf wenigstens der Mindesttemperatur gehalten. Gemäß Figur 1 ist das Durchgangsgehäuse 44 außenseitig von einer Heizeinrichtung 46 ummantelt, die beispielsweise wie bei der Mikrowellenkammer 16 ausgebildet ist. Die durch die Heizeinrichtung 46 eingebrachte Wärme verhindert ein Abkühlen des Granulats, so daß die in der Mikrowellenkammer 16 erzeugte Temperatur gehalten werden kann. Gegebenenfalls kann zwischen Eingang 43 und Ausgang 48 der Temperaturhaltekammer 17 ein Temperaturniveau eingestellte werden, wobei die Ein- und Ausgangstemperatur wenigstens eine für den Behandlungsvorgang erforderliche Mindesttemperatur aufweisen müssen. Zur Überwachung des Behandlungsvorganges werden die Einlauftemperatur über den Meßfühler 42 und die Ausgangstemperatur des Granulats in der Temperaturhaltekammer 17 über einen Meßfühler 47 erfaßt und dokumentiert.

Die Fördereinrichtung 45 besteht aus einem Förderschnecke, die ebenso wie die in der Mikrowellenkammer 16 wellenlos ausgebildet sein kann. Angetrieben wird die Förderschnecke 45 von einem Motor 49 mit steuerbarer Drehzahl. Ihre Drehzahl ist derart auf die Förderspirale 24 abgestimmt, daß es in der Temperaturhaltekammer 17 zu einer gewissen Verdichtung des für die Aufheizung durch Mikrowellen zunächst lockeren Granulats kommt, wodurch die Wärmeleitung in dieser Behandlungsphase verbessert und die Wärmeverluste verringert werden. Als Material für das Durchgangsgehäuse 44 und die Fördereinrichtung 45 sind Metalle vorgesehen, vorzugsweise Edelstahl. Es sind aber auch Kunststoffe oder keramische Materialien einsetzbar. Da am Ausgang 48 der Temperaturhaltekammer 17 der Behandlungsvorgang beendet ist, kann ein sich anschließender Ausförderabschnitt lediglich aus einer Auswurföffnung bestehen oder wie in Figur 1 dargestellt, eine seitlich aus dem Container 1 herausschwenkbare Ausförderschnecke 50, angetrieben von einem Motor 51, aufweisen.

Der in Figur 1 dargestellte Container 1 kann stationär aufgestellt werden oder gemäß Figur 1 für einen mobilen Einsatz auf einem Kraftfahrzeuganhänger 52 angeordnet sein. Eine Raumheizung 53 sorgt für eine ausreichende Umgebungstemperatur.

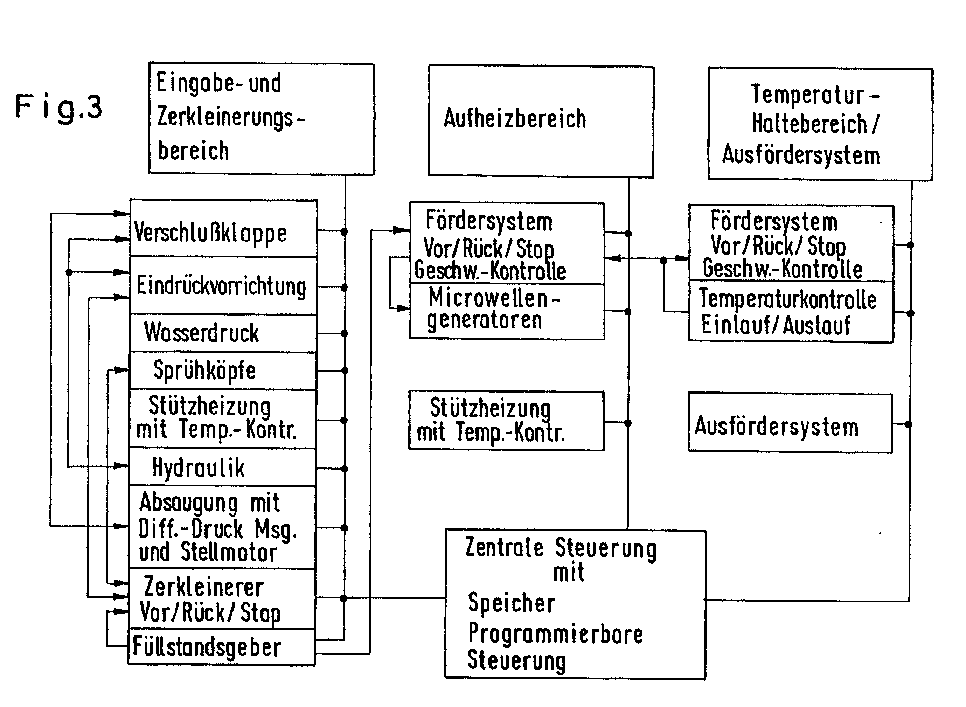

Die Bedienung der Entsorgungsanlage kann von Hand oder halb- bzw. vollautomatisch erfolgen. Alle wesentlichen Regelgrößen und Überwachungsfunktionen sind in einen Prozeßrechner 54 eingebunden. Kernstück des Prozeßrechners 54 ist eine speicherprogrammierte Steuerung, die ein Betriebsprogramm für Schalter- und Sensorabfragen, Motoren- und Sendersteuerung, Überwachung und Ansteuerung der Anzeigen enthält. Die an den Prozeßrechner 54 angeschlossenen Funktionseinheiten sind in einem Blockschaltbild der Figur 3 für den Eingabe- und Zerkleinerungsbereich, den Aufheizbereich und den Temperaturhaltebereich mit Ausfördersystem dargestellt. Die sich gegenseitig beeinflussenden Regelgrößen der Funktionseinheiten sind gekennzeichnet durch Verbindungslinien mit Pfeilen. Mit diesen Maßnahmen wird sichergestellt, daß die für eine thermische Behandlung, insbesondere eine Desinfektion, entscheidenden Größen Mindesttemperatur und Haltedauer eingehalten werden und Abweichungen automatisch nachgeregelt werden.

Die Funktionsweise der vorstehend beschriebenen Vorrichtung zur Behandlung von medizinischen Sonderabfällen wird nachfolgend für beispielhafte technische Daten zur rein thermischen Desinfizierung der Sonderabfälle beschrieben.

Zur Inbetriebnahme einer Anlage mit einer Verarbeitungskapazität von 100 bis 300 kg/h werden zunächst die Trogheizungen und die Trichterheizungen eingeschaltet. Ist die vorgegebene Solltemperatur erreicht, Wasser- und Luftdruck vorhanden und der Deckel der Eingabeschleuse geschlossen, kann die Anlage unter Beachtung der Sicherheitsbedingungen auf Automatikbetrieb geschaltet und beschickt werden.

Über die Hub- und Kippeinrichtung 12, die auch die Öffnung des Deckels 4 steuert, werden Abfälle aus den zu entleerenden Containern 13 in den geöffneten Trichter 3 geworfen. Nach dem Schließen des Deckels 4 wird die Absauganlage 8 abgestellt. Desweiteren werden die Drehschwinge 6 und der Zerkleinerer 7 in Betrieb gesetzt. Die Drehschwinge 6 zerreißt die Abfallgegenstände und führt sie gezielt dem Zerkleinerer 7 zu, der für eine Granulierung und Durchmischung sorgt. Das Granulat fällt dann in den Zwischentrog 18 mit automatischer Füllstandsüberwachung und gezielter Befeuchtung. Die Füllstandsüberwachung regelt den Zerkleinerungsvorgang, indem bei gefülltem Zwischentrog 18 der Zerkleinerer 17 abgeschaltet und bei leerem Zwischentrichter 18 der weitere Prozeßablauf gestoppt wird. Zeigt der untere Füllstandswächter 21 das Vorhandensein von Granulat an, so werden die Förderspiralen der Mikrowellenkammer 16 und der Temperaturhaltekammer 17 und zeitlich verzögert die Mikrowellensender 25 automatisch eingeschaltet. Die thermische Desinfektion wird nunmehr eingeleitet. Die Aufheizung des Granulats erfolgt in der Mikrowellenkammer bei einer hier für Industriezwecke zugelassenen Mikrowellenfrequenz von 2450 MHz. Das Granulat wird mit definierter Fördergeschwindigkeit durch die Mikrowellenkammer 16 bewegt, wo es zu einer schnellen, direkten Wärmeerzeugung im Material in Abhängigkeit von den dielektrischen Eigenschaften kommt. Dieser Effekt wird durch das zugegebene und unter Wirkung der Mikrowellen verdampfende Wasser noch gesteigert. Diese Dampfbeaufschlagung wird in der Mikrowellenkammer 16 aufrechterhalten, da ausgangsseitig ein natürlicher Verschluß durch das in die Temperaturhaltekammer 17 überführte Granulat aufgebaut wird. Die erreichte Granulattemperatur unterliegt einer Überwachung. Bei Unterschreiten einer Mindesttemperatur wird die Fördergeschwindigkeit herabgesetzt, bis die Mindesttemperatur wieder erreicht ist. Dabei ist die Drehzahl der Förderspirale 24 abgestimmt auf die zu erreichende mittlere Durchgangsleistung (kg/h) und die zu erreichende Mindesttemperatur.

Die für die Desinfektion abschließende Baugruppe ist die Temperaturhaltekammer 17 mit ihrer Haltestrecke. Auf dieser Haltestrecke wird das Granulat für die Ausschaltung der pathogenen Keime bei der mittels Mikrowellen erreichten Mindesttemperatur von über 95°C gehalten. Die Mindestverweildauer hängt ab von der Zahl der Keime, der Keimart und der Füllmenge. Die Haltedauer kann durch die Geschwindigkeit der Förderschnecke 45 gezielt angepaßt werden. Die Mindesttemperatur wird durch eine Dokumentation der automatisch gemessenen Einlauf- und Auslauftemperatur nachgewiesen. Temperaturverluste werden durch die Stützheizung ausgeglichen. Ist der Zwischentrichter 18 leer, so schalten sich zunächst die Mikrowellensender 25 und mit einem gewissen Nachlauf die Fördereinrichtungen automatisch ab. Das behandelte Granulat wird zwecks Abfuhr ausgeworfen. Nach dem Arbeitsende erfolgt eine Dampfdesinfektion der Anlage.

Ein erfindungsgemäßes Verfahren zur kontinuierlichen Wärmebehandlung von teilchenförmigen Gegenständen, insbesondere zur Desinfektion, Sterilisation oder Konservierung, umfaßt die folgenden zwei Schritte. In einem ersten Schritt werden die zerkleinerten oder bereits teilchenförmigen Gegenstände nach erfolgter Befeuchtung mit einem wäßrigen Medium aufgelockert und unter Mischung des gesamten Querschnitts der geförderten Schichtdicke durch ein Mikrowellenfeld bewegt und dabei auf eine Mindesttemperatur unter innerer Erhitzung aufgeheizt. In einem zweiten Schritt werden die so aufgeheizten Gegenstände zumindest leicht verdichtet und bei wenigstens der Mindesttemperatur während einer Mindestverweildauer gehalten. Zur Aufrechterhaltung dieser Mindesttemperatur bei auftretenden Wärmeverlusten kann eine indirekte Erwärmung der Gegenstände während dieser Haltephase vorgenommen werden, damit eine Unterschreitung der Mindesttemperatur vermieden wird. Hierdurch wird mit einer geringstmöglichen Einstrahlung von Mikrowellenenergie die zur Wärmebehandlung erforderliche Aufheizung sichergestellt und zudem für Folgeschritte genutzt, wodurch eine hohe Wirtschaftlichkeit gegeben ist. Dabei können die Gegenstände beispielsweise so lange auf der Mindesttemperatur gehalten werden, bis sie getrocknet sind. Sollte zusätzlich für einen Behandlungsvorgang der Einsatz von Desinfektionsmitteln erwünscht sein, so ist ein Einsprühen derselben während des ersten und/oder zweiten Schrittes möglich.

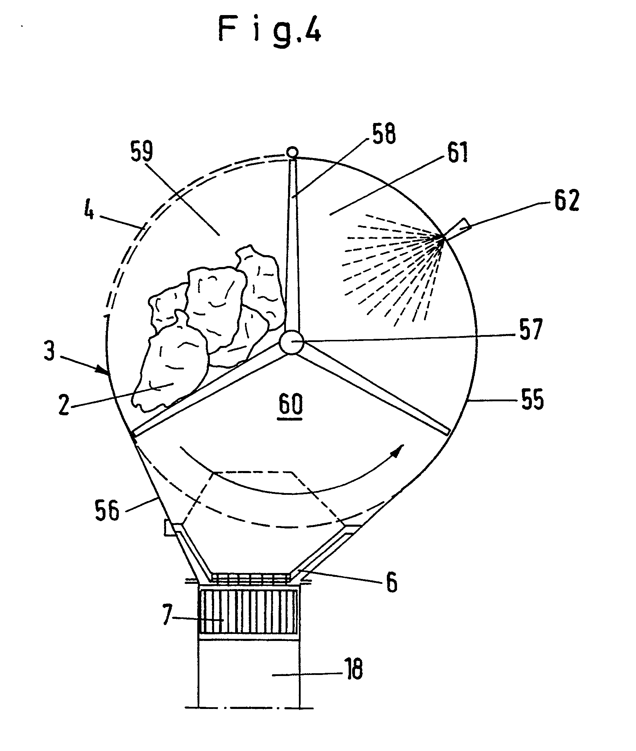

Figur 4 zeigt eine andere Ausführungsform des Eingabe- und Zerkleinerungsbereiches, insbesondere des Einfüllraumes 3, der als 3-Kammer-Schleuse ausgebildet ist. Der Einfüllraum besitzt einen im wesentlichen zylinderförmigen Hohlkörper 55, der bodenseitig mit einem trichterförmigen Ausgabeabschnitt 56 versehen ist. Mittig in dem Hohlkörper 55 ist ein Schleusenrad 57 mit drei unter jeweils einem Winkel von 120° sich auswärts erstreckenden Schleusenflügeln 58 angeordnet. Die Schleusenflügel 58 unterteilen den Hohlkörper 55 in drei voneinander getrennte Kammern, die bei Drehung des Schleusenrades 57 im Gegenuhrzeigersinn gemäß Pfeilrichtung umlaufen. Nacheinander durchlaufen die Schleusenkammern eine Einfüllstation 59, eine Übergabestation 60 und eine Desinfektionsstation 61. Für eine Kammer wird das Durchlaufen der drei Stationen 59, 60, 61 nachfolgend beschrieben. Befindet sich das Schleusenrad 58 in der in Figur 4 dargestellten Position, können nach Öffnen des Deckels 4 Abfälle 2 in die in der Einfüllstation sich befindenden Kammer eingefüllt werden. Durch Drehung um 120° wird diese Kammer mit den aufgenommenen Abfällen 2 in die Übergabestation 60 gedreht, wo die Drehschwinge 6 einwirkt und eine Übergabe an den Zerkleinerer 7 erfolgt. Die entleerte Kammer wird danach in die Desinfektionsstation 61 gebracht, die mit mindestens einem Sprühkopf 62 für ein Einbringen eines Desinfektionsnebels ausgestattet ist und mit einer Absauganlage 9 gemäß Figur 1 abgesaugt werden kann. Die von Keimen befreite erste Kammer wird dann wieder in die Einfüllstation verbracht. Die gegenüber dieser Kammer um 120° und 240° verdrehten Kammern durchlaufen diese Stationen 59, 60, 61 zeitlich versetzt, wodurch eine kontinuierliche Beschickung mit Abfällen 2 möglich ist. Der für eine Beschickung geöffnete Bereich des Einfüllraumes ist so stets keimfrei.

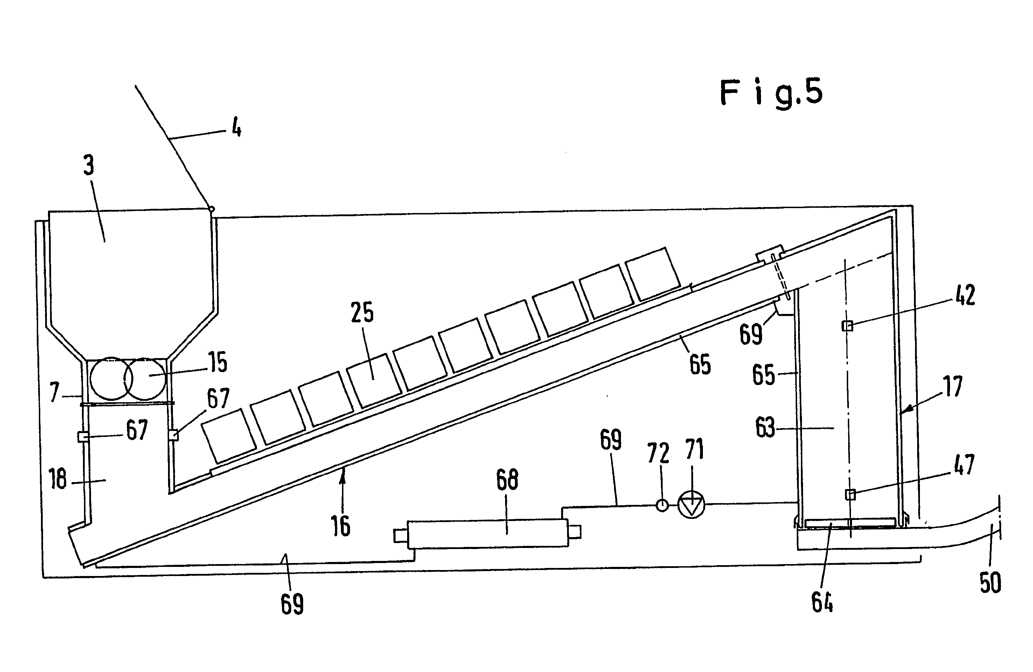

Figur 5 zeigt ein zweites Ausführungsbeispiel der Vorrichtung zum Behandeln von medizinischen Abfällen. Die Temperaturhaltekammer 17 ist hier als kompakter großvolumiger Behälter 63 ausgebildet. Dieser Behälter 63 ist im wesentlichen senkrecht aufgestellt, damit nach Inbetriebnahme der Anlage das in der Mikrowellenkammer 16 aufgeheizte Granulat unter Wirkung der Schwerkraft in den Behälter 63 hinabfällt und diesen füllt. Um zu verhindern, daß bei dem ersten Füllen des Behälters 63 noch nicht ausreichend thermisch behandeltes Gut austritt, ist der Behälter 63 bodenseitig mit einem entfernbaren Deckel 64 verschlossen. Das in den Behälter 63 eingefüllte Granulat verbleibt in diesem für eine Mindestverweildauer bei wenigstens der Mindesttemperatur. In Abhängigkeit von der gewünschten thermischen Behandlung, z.B. Desinfektion, sind die Mindesttemperatur und die Mindestverweildauer entsprechend einstellbar und über die Temperaturmeßfühler 42, 47 kontrollierbar. Für einen kontinuierlichen Betrieb wird nach einem ersten Füllen des Behälters 63 der Deckel 64 entfernt. Das von der Fördereinrichtung der Mikrowellenkammer 16 an die Temperaturhaltekammer übergebene Granulat versetzt das bereits in den Behälter 63 eingefüllte Granulat in eine Durchgangsbewegung. Die Abmessungen des Behälters 63 sind an die Durchsatzmenge der Anlage anpaßbar. Der Querschnitt der an den Boden des Behälters 63 angeschlossenen Ausfördereinrichtung 50 ist kleiner ausgebildet als der Querschnitt des Förderkanals der Mikrowellenkammer 16, so daß ständig mehr Granulat von der Mikrowellenkammer 16 an den Behälter 63 übergeben wird als von dieser über die Ausfördereinrichtung 50 abgegeben werden kann, wodurch eine Verdichtung des Granulats in dem Behälter 63 erreicht wird.

Die zweite Wäremeinspeisung für die Mikrowellenkammer 16 und eine indirekte Beheizung des Granulats in dem Behälter 63 erfolgt bei der Anlage gemäß Figur 5 über ein Wärmeträgermedium. Die Mikrowellenkammer 16 und der Behälter 63 besitzen hierfür Kammerwandungen mit einem Doppelmantel 65, durch die das Wärmeträgermedium, z.B. Thermoöl, Heißdampf, aus einem Vorratsbehälter 68 gepumpt wird. Die Doppelmäntel 65 der Mikrowellenkammer 16 und des Behälters 63 sind zur Ausbildung eines Kreislaufes für das Wärmeträgermedium über Leitungen 69 miteinander verbunden, in denen zur Beaufschlagung und Steuerung eine Pumpe 71 und mindestens ein Ventil 72 vorgesehen sind. Von diesem Wärmeträgerkreislauf kann auch die Heizeinrichtung des Einfülltrichters 3 gespeist werden. Mittels Temperaturfühler 67 wird die so erreichte Vorwärmung des Granulats in dem Zwischentrichter 18 überprüft. Im übrigen kann die Anlage, wie zu den Figuren 1 bis 4 beschrieben, ausgebildet sein und auch mit einer elektrischen Heizung bestückt sein.

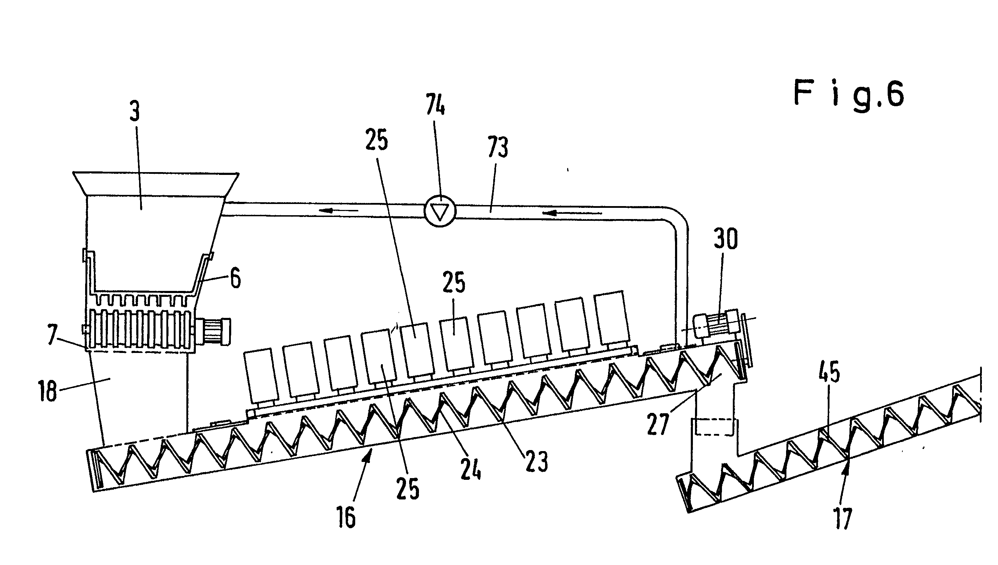

Figur 6 zeigt ein drittes Ausführungsbeispiel einer Vorrichtung zum Behandeln von medizinischen Sonderabfällen, bei dem die bei der Mikrowellenbestrahlung der befeuchteten Abfälle entstehende Wärme zumindest teilweise rückgewonnen werden kann. Hierzu ist im Bereich des Ausgangs 27 der Mikrowellenkammer 16 eine absperrbare Umluftleitung 73 angeschlossen, die in den Einfüllraum 3 geführt ist. Mittels einer in die Umluftleitung 73 eingesetzten Pumpe 74 kann Warmluft aus dem Gehäusekanal 23 der Mikrowellenkammer 16 abgesaugt und dem trichterförmigen Einfüllraum 3 zugeführt werden. Ansonsten unterscheidet sich diese Anlage von der in Figur 1 beschriebenen nicht.

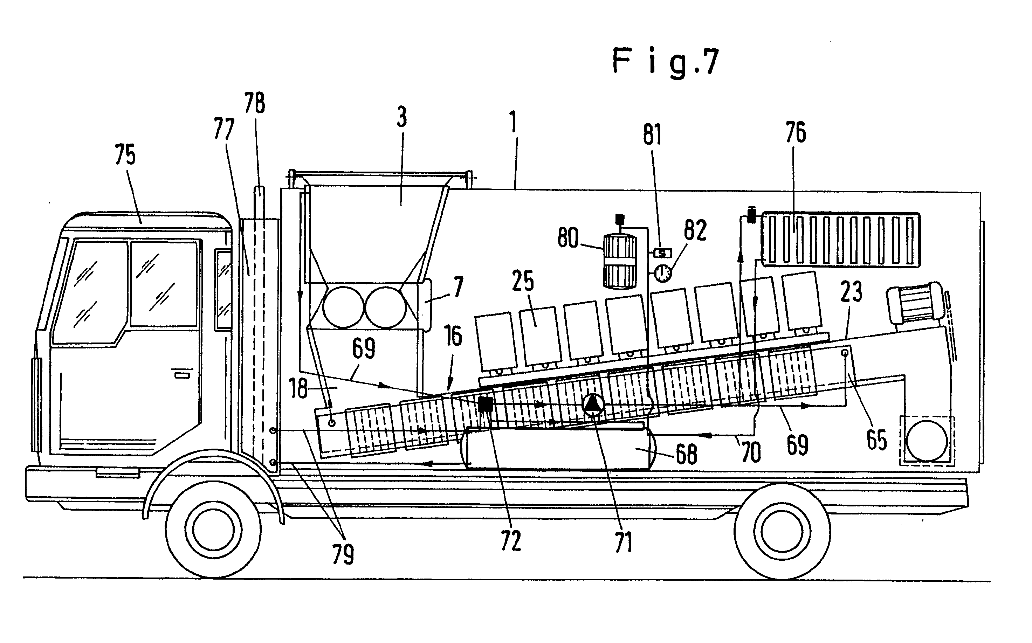

Figur 7 zeigt eine mobile Ausführungsform einer Vorrichtung zum Behandeln von medizinischen Abfällen. Wie zu Figur 1 beschrieben umfaßt diese Anlage einen Eingabeabschnitt mit Einfüllraum 3, Zerkleinerer 7 sowie Zwischentrichter 18, einen Behandlungsabschnitt mit Mikrowellenkammer 16 sowie Temperaturhaltekammer und einen Ausförderabschnitt, wobei diese Betriebseinheiten in einem Container 1 der den Aufbau eines Kraftfahrzeuges 75 bildet, untergebracht sind. Zur Vereinfachung der zeichnerischen Darstellung sind die Temperaturhaltekammer und der Ausörderabschnitt weggelassen worden. Desweiteren ist die Anlage mit einer zu Figur 5 beschriebenen Heizeinrichtung mittels Kreislauf eines Wärmeträgeröls ausgestattet. Als Wärmeträgermedium ist hier Wärmeträgeröl vorgesehen. Leitungen 69 mit eingesetzter Pumpe 71 und Ventil 72 verbinden den Vorratsbehälter 68 mir den doppelwandigen Mänteln 65 des Gehäusekanals 23 der Mikrowellenkammer 16 und des Trichters 3. Über Leitungen 70 wird ein Heizkörper 76 der Raumheizung 53 (siehe Figur 1) gespeist. Zur Aufheizung des in dem Vorratsbehälter 68 gelagerten Wärmeträgeröls ist ein Abgaswärmetauscher 77 vorgesehen, der um den Auspuff 78 des Kraftfahrzeuges 75 angeordnet ist. Über Leitungen 79 ist der Vorratsbehälter 68 mit dem Abgaswärmetauscher 77 zur Aufheizung des Wärmeträgermediums verbunden. Zur Überwachung und Sicherung der Aufheizung des Wärmeträgeröls sind an den Vorratsbehälter 68 ein Ausdehnungsgefäß 80, ein Sicherheitsventil 81 und ein Manometer 82 angeschlossen. Die in der Verbrennungsmaschine des Kraftfahrzeuges erzeugte Wärme kann so für den thermischen Behandlungsvorgang des medizinischen Sondermülls in der Weise genutzt werden, daß das Aufheizen der Anlage während der Anfahrt zu den Einsatzorten ohne zusätzliche Energiekosten erfolgt und Vorheizzeiten bzw. Standzeiten der Anlage minimiert werden.

## Patentansprüche

1. Vorrichtung zum Behandeln von feuchtem, gegebenenfalls zu befeuchtendem, infektiösem Müll mit Mikrowellen in Form einer nach außen verschließbaren Baueinheit, bestehend aus einem Eingabeabschnitt mit einem Einfüllraum und einem Zerkleinerer, und einem sich daran anschließenden Behandlungsabschnitt mit einer Mikrowellenkammer, die eine Transporteinrichtung für eine Bewegung des Mülls entlang mehreren in Durchlaufrichtung nebeneinander angeordneten Mikrowellensendern aufweist, dadurch gekennzeichnet, daß der Behandlungsabschnitt zweistufig ausgebildet ist mit einer ersten Durchgangskammer, die gebildet wird von einer Mikrowellenkammer (16) mit einer in Durchgangsrichtung dichten Mikrowellenfeldverteilung zum Aufheizen des Mülls auf oder über eine wählbare Mindesttemperatur, und mit einer zweiten Durchgangskammer, die an den Ausgang (27) der ersten Durchlaufkammer angeschlossen und als Temperaturhaltekammer (17) zum Halten des Mülls auf wenigstens der Mindesttemperatur während einer Mindestverweildauer ausgebildet ist.

2. Vorrichtung nach Anspruch 1, dadurch gekennzeichnet, daß die Temperaturhaltekammer (17) eine Heizeinrichtung (46) zur indirekten Erwärmung des Mülls aufweist.

3. Vorrichtung nach Anspruch 2, dadurch gekennzeichnet, daß die Heizeinrichtung (46) elektrisch, mit Thermoöl oder mit Heißdampf betreibbar ist.

4. Vorrichtung nach einem der Ansprüche 1 bis 3, dadurch gekennzeichnet, daß die Temperaturhaltekammer (17) von einem Gehäusekanal (44) gebildet wird, in dem eine Förderspirale angeordnet ist.

5. Vorrichtung nach einem der Ansprüche 1 bis 3, dadurch gekennzeichnet, daß die Temperaturhaltekammer (17) aus einem Lagerbehälter (63) mit einem wählbaren Querschnitt für eine Mindestfüllmenge besteht, der bodenseitig eine Ausgangsöffnung für eine Ausgabe des Mülls unter Wirkung der Schwerkraft besitzt.

6. Vorrichtung nach Anspruch 5, dadurch gekennzeichnet daß zur mindestens teilweisen Verdichtung des Mülls in der Temperaturhaltekammer (17) ein Gehäusekanal (23) der Mikrowellenkammer (16) einen größeren Durchmesser aufweist als ein Förderkanal der Ausfördereinrichtung (50).

7. Vorrichtung nach einem der Ansprüche 1 bis 6, dadurch gekennzeichnet, daß die Temperaturhaltekammer (17) als Ausfördereinrichtung ausgebildet ist.

8. Vorrichtung nach einem der Ansprüche 1 bis 7, dadurch gekennzeichnet, das die Mikrowellenkammer (16) mit einem Gehäusekanal (23) ausgestattet ist, in dem eine als Mikrowellenfeldverteiler arbeitende Fördereinrichtung (24) angeordnet ist.

9. Vorrichtung nach Anspruch 8, dadurch gekennzeichnet, daß die Fördereinrichtung (24) als wellenlose Förderschnecke aus Metall, insbesondere Edelstahl, ausgebildet ist.

10. Vorrichtung nach einem der Ansprüche 1 bis 9, dadurch gekennzeichnet, daß die Mikrowellenkammer (16) aus einem metallischen U-förmigen Trog mit aufsetzbarem Trogdeckel (29) besteht, an dem die Mikrowellensender (25) befestigt sind.

11. Vorrichtung nach Anspruch 10, dadurch gekennzeichnet, daß der Trog von den Mikrowellensendern (25) mittels einer Scheibe aus Polytetrafluorethylen (PTFE) fluiddicht abgetrennt ist.

12. Vorrichtung nach Anspruch 10 oder 11, dadurch gekennzeichnet daß der Trog mit einer außenseitig angeordneten Heizeinrichtung zur Erwärmung der Trogwandungen ausgestattet ist.

13. Vorrichtung nach Anspruch 12, dadurch gekennzeichnet, daß die Heizeinrichtung von einen Doppelmantel (38) für ein Wärmeträgermedium oder von elektrischen Heizelementen (40)und einer darum angeordneten Isolierschicht (39) gebildet wird.

14. Vorrichtung nach einem der Ansprüche 1 bis 13, dadurch gekennzeichnet, daß die Mikrowellensender (25) über jeweils einen Hohlleiter (31) mit angesetztem Resonanzraum (33) an der Mikrowellenkammer (16) befestigt sind, wobei die Resonanzräume (33) im wesentlichen ohne Abstand benachbart zueinander angeordnet sind.

15. Vorrichtung nach einem der Ansprüche 1 bis 14, dadurch gekennzeichnet, daß zwischen dem Zerkleiner (7) und der Mikrowellenkammer (16) ein Übergabetrichter (18) vorgesehen ist.

16. Vorrichtung nach Anspruch 15, dadurch gekennzeichnet, daß Eingang und Ausgang des Übergabetrichters (18) jeweils mit einem Füllstandswächter (21) ausgestattet sind.

17. Vorrichtung nach einem der Ansprüche 1 bis 16, dadurch gekennzeichnet, daß der Einfüllraum (3) als trichterförmige Einfüllschleuse mit beheizbaren Trichterwänden und einem abdichtbaren Deckel (4) ausgebildet ist.

18. Vorrichtung nach einem der Ansprüche 1 bis 17, dadurch gekennzeichnet, daß der Einfüllraum (3) an eine Absauganlage (9) angeschlossen ist, die die gegebenenfalls mit Keimen behaftete Luft aus dem Einfüllraum (3) vor einem Öffnen desselben entfernt.

19. Vorrichtung nach einem der Ansprüche 1 bis 18, dadurch gekennzeichnet, daß die Vorrichtung als Aufbau eines Kraftfahrzeuges (75) mobil ausgebildet ist.

20. Vorrichtung nach Anspruch 19, dadurch gekennzeichnet, daß ein Abgaswärmetauscher (77) zur Erwärmung des Wärmeträgemediums vorgesehen ist.

21. Vorrichtung nach einem der Ansprüche 1 bis 20, dadurch gekennzeichnet, daß der Einfüllraum (3) als Mehrkammerschleuse (59, 60, 61) mit einem umlaufenden Schleusenrad (57) ausgebildet ist.

22. Vorrichtung nach einem der Ansprüche 1 bis 21, dadurch gekennzeichnet, daß eine Meß- und Regeleinrichtung (54) für die Bestimmung der Durchlaufgeschwindigkeit in Abhängigkeit von der Mindesttemperatur vorgesehen ist, an die die Füllstandsmeßfühler des Übergabebereiches, die Temperaturfühler der Temperaturhaltekammer und Antriebseinrichtungen für die Fördereinrichtung in der Mikrowellenkammer und gegebenenfalls der Temperaturhaltekammer angeschlossen sind.

23. Verfahren zur kontinuierlichen Wärmebehandlung teilchenförmiger Gegenstände, insbesondere zur Desinfektion, Sterilisation oder Konservierung, bei dem die Gegenstände zunächst befeuchtet und dann durch ein Mikrowellenfeld gefördert werden, dessen Einwirkungszeit lang genug ist, die Wärmebehandlung durchzuführen, dadurch gekennzeichnet, daß die Gegenstände in einem ersten Schritt aufgelockert und unter Mischung durch das Mikrowellenfeld geführt und dabei auf eine Mindesttemperatur unter innerer Erwärmung aufgeheizt werden und in einem zweiten Schritt zumindest leicht verdichtet und bei wenigstens der Mindesttemperatur während einer Mindestverweildauer gehalten werden.

24. Verfahren nach Anspruch 23, dadurch gekennzeichnet, daß zur Desinfektion von medizinischen Sondermüll die Mindesttemperatur zwischen 98°C und 102°C liegt.

## Claims

1. Apparatus for treating moist, possibly to be moistened, infectious refuse with microwaves in the form of an outwardly sealable structural unit comprising a loading section having a loading chamber and a comminutor, a treatment section, adjoining thereto, having a microwave chamber which incorporates a conveying device for moving the refuse past a plurality of microwave sources arranged next to each other in the direction of travel, characterised in that the treatment section is of two-stage construction, having a first through chamber which is formed by a microwave chamber (16) having a dense microwave field distribution in the direction of travel for heating up the refuse to or above a selectable minimum temperature, and having a second through chamber which is connected to the output (27) of the first through chamber and is constructed as temperature maintenance chamber (17) for maintaining the refuse at at least the minimum temperature during a minimum residence time.

2. Apparatus according to Claim 1, characterised in that the temperature maintenance chamber (17) incorporates a heating device (46) for indirectly heating the refuse.

3. Apparatus according to Claim 2, characterised in that the heating device (46) can be operated electrically, with thermal oil or with superheated steam.

4. Apparatus according to any of Claims 1 to 3, characterised in that the temperature maintenance chamber (17) is formed by a housing duct (44) in which a conveying helix is disposed.

5. Apparatus according to any of Claims 1 to 3, characterised in that the temperature maintenance chamber (17) comprises a storage container (63) with a selectable cross-section for a minimum filling quantity, which container has an outlet opening at the bottom for discharging the refuse under the action of gravity.

6. Apparatus according to Claim 5, characterised in that a housing duct (23) of the microwave chamber (16) has a larger diameter than a conveying duct of the unloading device (50) in order to at least partially compact the refuse in the temperature maintenance chamber (17).

7. Apparatus according to any of Claims 1 to 6, characterised in that the temperature maintenance chamber (17) is constructed as unloading device.

8. Apparatus according to any of Claims 1 to 7, characterised in that the microwave chamber (16) is equipped with a housing duct (23) in which a conveying device (24) is disposed which operates as microwave field distributor.

9. Apparatus according to Claim 8, characterised in that the conveying device (24) is constructed as a shaftless conveying screw made of metal, in particular stainless steel.

10. Apparatus according to any of Claims 1 to 9, characterised in that the microwave chamber (16) comprises a metallic U-shaped trough with mountable trough cover (29) to which the microwave sources (25) are attached.

11. Apparatus according to Claim 10, characterised in that the trough is separated from the microwave sources (25) by means of a sheet of polytetrafluoroethylene (PTFE) in a fluid-tight manner.

12. Apparatus according to either of Claims 10 or 11, characterised in that the trough is equipped with a heating device, disposed on the outside, for heating the trough walls.

13. Apparatus according to Claim 12, characterised in that the heating device is formed by a double jacket (38) for a heat transfer medium or by electrical heating elements (40) and an insulating layer (39) disposed around the jacket or elements.

14. Apparatus according to any of Claims 1 to 13, characterised in that the microwave sources (25) are attached to the microwave chamber (16) via a waveguide (31) in each case with a resonance cavity (33) attached, the resonance cavities (33) being disposed adjacent to each other essentially without spacing.

15. Apparatus according to any of Claims 1 to 14, characterised in that a transfer funnel (18) is provided between the comminutor (7) and the microwave chamber (16).

16. Apparatus according to Claim 15, characterised in that the inlet and outlet of the transfer funnel (18) are each equipped with a filling level sensor (21).

17. Apparatus according to any of Claims 1 to 16, characterised in that the loading chamber (3) is constructed as a funnel-type loading sluice with heatable funnel walls and a sealable cover (4).

18. Apparatus according to any of Claims 1 to 17, characterised in that the loading chamber (3) is connected to a suction system (9) which removes the air, which is possibly encumbered with germs, from the loading chamber (3) before the same is opened.

19. Apparatus according to any of Claims 1 to 18, characterised in that the apparatus is of mobile construction as the superstructure of a motor vehicle (75).

20. Apparatus according to Claim 19, characterised in that an exhaust gas exchanger (77) is provided to heat the heat transfer medium.

21. Apparatus according to any of Claims 1 to 20, characterised in that the loading chamber (3) is constructed as a multi-chamber sluice (59, 60, 61) with a rotating sluice wheel (57).

22. Apparatus according to any of Claims 1 to 21, characterised in that a measuring and regulating device (54) is provided for determining the speed of travel as a function of the minimum temperature, to which device the filling level measuring sensor of the transfer region, the temperature sensor of the temperature maintenance chamber and drive devices for the conveying device in the microwave chamber and possibly the temperature maintenance chamber are connected.

23. Process for continuously heat-treating particulate articles, in particular for disinfecting, sterilising or preserving, in which the articles are first moistened and then passed through a microwave field whose processing time is long enough to carry out the heat treatment, characterised in that the articles are loosened up in a first step and passed through the microwave field while being mixed and are heated during this process to a minimum temperature with internal heating, and are at least slightly compacted in a second step and are held at at least the minimum temperature during a minimum residence time.

24. Process according to Claim 23, characterised in that the minimum temperature for disinfecting medical hazardous refuse is between 98°C and 102°C.

## Revendications

1. Dispositif pour le traitement par micro-ondes de déchets infectés, humides, le cas échéant à humidifier, sous la forme d'une unité que l'on peut fermer vis-à-vis de l'extérieur, composé d'une section d'entrée munie d'un compartiment de chargement et d'un concasseur, et une section de traitement, raccordée à ce dernier, munie d'une chambre à micro-ondes qui comporte un dispositif transporteur mettant en mouvement des déchets le long de plusieurs émetteurs de micro-ondes disposés les uns à côté des autres dans la direction de passage, caractérisé en ce que la section de traitement est formée de deux étages, à savoir, une première chambre de passage qui est formée d'une chambre à micro-ondes (16), avec une répartition du champ de micro-ondes dense dans la direction de passage pour chauffer les déchets à une température minimum sélectionnable ou au-dessus de cette dernière, et une deuxième chambre de passage qui est reliée à la sortie (27) de la première chambre de passage et qui est réalisée pour former une chambre de maintien de la température (17) qui maintient les déchets à la température minimum au moins pendant une durée d'arrêt minimum.

2. Dispositif selon la revendication 1, caractérisé en ce que la chambre de maintien de la température (17) présente un dispositif de chauffage (46) pour le chauffage indirect des déchets.

3. Dispositif selon la revendication 2, caractérisé en ce que le dispositif de chauffage (46) peut fonctionner électriquement, au mazout ou à la vapeur chaude.

4. Dispositif selon l'une des revendications 1 à 3, caractérisé en ce que la chambre de maintien de la température (17) est formée d'un carter en forme de canal (44) dans lequel est disposé un transporteur à spirale.

5. Dispositif selon l'une des revendications 1 à 3, caractérisé en ce que la chambre de maintien de la température (17) est constituée d'un conteneur de stockage (63) dont on peut choisir la section pour un chargement minimum, ledit conteneur présentant, du côté du fond, une ouverture de sortie pour une distribution des déchets sous l'effet de la pesanteur.

6. Dispositif selon la revendication 5, caractérisé en ce que, pour comprimer au moins partiellement les déchets dans la chambre de maintien de la température (17), un carter en forme de canal (23) de la chambre de micro-ondes (16) présente un diamètre plus grand que le canal de transport du dispositif extracteur (50).

7. Dispositif selon l'une des revendications 1 à 6, caractérisé en ce que la chambre de maintien de la température (17) est réalisée pour former un dispositif extracteur.

8. Dispositif selon l'une des revendications 1 à 7, caractérisé en ce que la chambre à micro-ondes (16) est munie d'un carter en forme de canal (23) dans lequel est installé un dispositif transporteur (24) fonctionnant comme répartiteur de champ de micro-ondes.

9. Dispositif selon la revendication 8, caractérisé en ce que le dispositif transporteur (24) a la forme d'une vis sans fin sans axe en métal, en particulier en métal noble.

10. Dispositif selon l'une des revendications 1 à 9, caractérisé en ce que la chambre à micro-ondes (16) a la forme d'un bac métallique en forme de U muni d'un couvercle (29) sur lequel sont fixés les émetteurs de micro-ondes (25).

11. Dispositif selon la revendication 10, caractérisé en ce que la bac est séparé des émetteurs de micro-ondes (25) de façon étanche au moyen d'une plaque de polytétrafluoroéthylène (PTFE).

12. Dispositif selon la revendication 10 ou 11, caractérisé en ce que le bac est équipé d'un dispositif de chauffage disposé sur un côté extérieur pour le chauffage des parois du bac.

13. Dispositif selon la revendication 12, caractérisé en ce que le dispositif de chauffage est formé d'une double paroi (38) pour un milieu caloporteur ou d'éléments de chauffage électriques (40) et d'une couche isolante (39) disposée autour de ces derniers.

14. Dispositif selon l'une des revendications 1 à 13, caractérisé en ce que les émetteurs de micro-ondes (25) sont fixés chacun à la chambre à micro-ondes (16) par l'intermédiaire d'un tube guide d'ondes (31) muni d'une cavité résonnante rapportée (33), les cavités résonnantes (33) étant disposées les unes à côté des autres, en substance sans espace entre elles.

15. Dispositif selon l'une des revendications 1 à 14, caractérisé en ce qu'une trémie de transfert (18) est prévue entre le concasseur (7) et la chambre à micro-ondes (16).

16. Dispositif selon la revendication 15, caractérisé en ce que l'entrée et la sortie de la trémie de transfert (18) sont chacune munie d'un dispositif du contrôle (21) de l'état du chargement.

17. Dispositif selon l'une des revendications 1 à 16, caractérisé en ce que le compartiment de chargement (3) à la configuration d'un sas de chargement en forme de trémie muni de parois de trémie que l'on peut chauffer et d'un couvercle (4) que l'on peut étancher.

18. Dispositif selon l'une des revendications 1 à 17, caractérisé en ce que le compartiment de chargement (3) est relié à une installation d'aspiration (9) qui aspire hors du compartiment de chargement (3) l'air éventuellement chargé de germes avant une ouverture de ce compartiment.

19. Dispositif selon l'une des revendications 1 à 18, caractérisé en ce que le dispositif est réalisé sous forme d'un véhicule (75) automobile.

20. Dispositif selon la revendication 19, caractérisé en ce qu'un échangeur de chaleur avec l'échappement (77) est prévu pour le chauffage du milieu caloporteur.

21. Dispositif selon l'une des revendications 1 à 20, caractérisé en ce que le compartiment de chargement (3) est réalisé sous la forme d'un sas à plusieurs chambres (59, 60, 61) avec une roue (57) à sas tournante.

22. Dispositif selon l'une des revendications 1 à 21, caractérisé en ce qu'il est prévu un dispositif de mesure et de réglage (54) pour la détermination de la vitesse de passage en liaison avec la température minimum auquel sont reliés les détecteurs de mesure de l'état de chargement de la zone de transfert, les détecteurs de température de la chambre de maintien de température et des dispositifs d'entraînement du dispositif transporteur dans la chambre à micro-ondes et le cas échéant de la chambre de maintien de la température.

23. Procédé pour traiter à chaud et de façon continue des objets en forme de particules, en particulier pour la desinfection, la stérilisation ou la conservation selon lequel les objets sont tout d'abord humidifiés et ensuite transportés à travers un champ de micro-ondes dont le temps d'action est suffisamment long pour réaliser le traitement à chaud, caractérisé en ce que les objets sont, dans une première étape, dispersés, et conduits en étant mélangés dans un champ de micro-ondes et donc chauffés à une température minimum par un chauffage interne, et, dans une deuxième étape, au moins légèrement comprimés et maintenus au moins à la température minimum pendant un temps d'arrêt minimum.

24. Procédé selon la revendication 23, caractérisé en ce que pour la désinfection de déchets médicaux spéciaux, la température minimum est située entre 98°C et 102°C.

Fig.1

# Fig.2a     Fig.2b

Fig.3

| Eingabe- und Zerkleinerungs- bereich | Aufheizbereich | Temperatur- Haltebereich/ Ausfördersystem |

**Eingabe- und Zerkleinerungsbereich**
- Verschlußklappe
- Eindrückvorrichtung
- Wasserdruck
- Sprühköpfe
- Stützheizung mit Temp.-Kontr.
- Hydraulik
- Absaugung mit Diff.-Druck Msg. und Stellmotor
- Zerkleinerer Vor/Rück/Stop
- Füllstandsgeber

**Aufheizbereich**
- Fördersystem Vor/Rück/Stop Geschw.-Kontrolle
- Microwellen- generatoren
- Stützheizung mit Temp.-Kontr.

**Temperatur-Haltebereich/Ausfördersystem**
- Fördersystem Vor/Rück/Stop Geschw.-Kontrolle
- Temperaturkontrolle Einlauf/Auslauf
- Ausfördersystem

**Zentrale Steuerung mit Speicher Programmierbare Steuerung**

EP 0 393 231 B1

# Fig.4

Fig.5

EP 0 393 231 B1

Fig.6

Fig.7